# FASCICULE DE BREVET EUROPEEN

(11) **EP 0 605 701 B1**
(45) Date de publication et mention de la délivrance du brevet: **07.10.1998**
(21) Numéro de dépôt: 93916038.8
(22) Date de dépôt: 26.07.1993
(51) Int. Cl.: G01N 1/30, A61K 31/475, G01N 33/50

(54) **METHODE DE CYTODIAGNOSTIC UTILISANT L'ALSTONINE COMME MARQUEUR SELECTIF ET KIT DE DIAGNOSTIC CONTENANT CE MARQUEUR**
ZYTODIAGNOSTISCHES VERFAHREN UNTER VERWENDUNG VON ALSTONIN ALS SELEKTIVEM MARKER UND DIESEN MARKER ENTHALTENDER DIAGNOSTISCHER KIT
CYTODIAGNOSIS METHOD USING ALSTONINE AS A SELECTIVE MARKER, AND DIAGNOSTIC KIT CONTAINING SAID MARKER

(30) Priorité: 28.07.1992 FR 9209283
(43) Date de publication de la demande: 13.07.1994
(73) Titulaire: Molecular International Research, Inc., New York, NY 10022-6501 (US)
(72) Inventeur: BELJANSKI, Mirko, F-75005 Paris (FR)
(74) Mandataire: Leboyer, Jean-Jacques
(86) Numéro de dépôt international: FR9300762
(87) Numéro de publication internationale: WO9402829

(56) Documents cités:
- EP-A- 0 059 817
- FR-A- 2 419 725
- FR-A- 2 439 783
- IRCS MEDICAL SCIENCE, Vol. 12, 1984, pages 587-588, M. BELJANSKI et al., "Three Alkaloids As Selective Destroyers of the Proliferative Capacity of Cancer Cells".
- ONCOLOGY, Vol. 43, 1986, pages 198-203, M. BELJANSKI et al., "Three Alkaloids as Selective Destroyers of Cancer Cells in Mice".

## Description

La présente invention concerne les techniques de cytodiagnostic.

Elle concerne plus particulièrement la détection des tumeurs, notamment pour l'établissement d'un diagnostic tumoral, et celle des aberrations chromosomiques, au moyen d'alstonine avec un procédé de révélation sélective par fluorescence.

Dans la présente description on entend par cytodiagnostic aussi bien le diagnostic des affections tumorales que la cytogénétique.

L'alstonine est un alcaloïde extrait de Rauwolfia vomitoria, Rauwolfia obscura et Vinca rosea, ainsi que d'autres apocynacées. Elle appartient au groupe des bêta-carbolines quaternaires. Des procédés d'extraction de cet alcaloïde ont été décrits par M. Beljanski et J. Bugiel dans les documents FR-A-78 30663 et EP-A-0 059 817, dont le contenu est incorporé ici par référence. Il a également été montré que cet alcaloïde présente une fluorescence très vive et bleu pâle, correspondant par analyse au moyen d'un spectrophotomètre à deux maxima d'absorption, respectivement à 252 et à 308 nm, caractéristiques de l'alstonine et de son isomère, la serpentine.

Il a également été montré, notamment dans les documents susdits, que l'alstonine possède des propriétés anti-cancéreuses désormais bien établies sur les cellules ascitiques du lymphome YC8 de la souris, sur des tumeurs "ascites" d'Ehrlich chez la souris, sur des cellules cancéreuses en culture de type KB, HELA, HEPII et L et sur des cellules provenant de néphro-blastome, de neuro-blastome ou de tératome, qui sont toutes des tumeurs humaines en cultures primaires dont l'alstonine s'est avérée assurer la destruction totale en 24/48 heures. A titre de comparaison, afin de bien établir la sélectivité de l'action de l'alstonine à cet égard, la même dose d'alstonine (200 µg par ml de culture) a été appliquée à des cultures de cellules primaires de reins de singe ou sur des cellules de lignées de type Véro ou Circ; elle n'a produit dans les 8 jours aucune destruction cellulaire. Cela attestait de l'interaction spécifique de l'alstonine avec l'ADN des cellules tumorales, alors que l'ADN des cellules normales n'était pas affecté par cet alcaloïde.

Il était cependant encore délicat de bien maîtriser un traitement antitumoral utilisant un tel alcaloïde et/ou de pouvoir donner un diagnostic sérieux de l'état d'un patient aux stades pré-, per- et postopératoires.

Il existait donc un besoin pour un moyen d'évaluation des affections tumorales destiné à faciliter le diagnostic des lésions cancéreuses et des métastases et à en améliorer le traitement.

On a maintenant trouvé de manière inattendue que l'alstonine elle-même, utilisée en une concentration appropriée dans des compositions mises sous une forme convenant à des applications in vitro ou in vivo, permet, par révélation appropriée de sa bioluminescence après son incubation, d'obtenir un diagnostic sélectif des affections tumorales,tant pour ce qui concerne leur localisation que pour l'appréciation de leur ampleur. On a également trouvé qu'une composition d'alstonine analogue est utile en cytogénétique. L'alstonine s'est en effet avérée présenter un spectre de fluorescence centré sur la longueur d'onde 446 nm, tandis que son spectre d'excitation de la fluorescence et son spectre d'absorption présentaient tous deux des maxima au nombre de trois, correspondant respectivement aux longueurs d'onde de 252 nm, 310 nm et 375 nm.

L'invention a donc pour premier objet l'utilisation d'une composition d'alstonine et/ou d'un isomère de celle-ci comme marqueur sélectif des cellules tumorales et/ou des aberrations chromosomiques, pour la révélation de celles-ci par mesure de fluorescence à environ 375 nm.

L'invention a également pour objet l'utilisation d'une composition d'alstonine pour l'obtention d'un réactif de cytodiagnostic destiné à la révélation sélective des affections tumorales et/ou des aberrations chromosomiques.

Elle a encore pour objet une méthode d'évaluation des tumeurs et/ou de cytogénétique, utile pour le diagnostic des affections tumorales et des aberrations chromosomiques, comprenant la révélation des cellules tumorales par fluorescence sélective détectée au moyen d'une activation par une lampe de Wood émettant à environ 375 nm ou d'une détection au microscope à fluorescence à environ 375 nm, après fixation sélective d'une quantité appropriée d'une telle composition pharmaceutique et incubation.

Un autre objet de l'invention est un kit ou une trousse pour faciliter le diagnostic des affections tumorales et/ou des aberrations chromosomiques, comportant notamment une composition comprenant de l'alstonine sous une forme galénique appropriée pour l'utilisation susmentionnée.

On a en effet pu établir qu'une solution d'alstonine à 5.10⁻⁶ M excitée aux trois longueurs d'ondes de 260, 310 et 375 nm donne une fluorescence dans le domaine du visible (446 nm) avec des rendements quantiques différents d'un maximum à l'autre. L'émission la plus forte correspond au maximum à 310 nm.

Le spectre d'absorption dans l'U.V. a donné trois bandes d'absorption vers respectivement 260, 310 et 320 nm.

Le rendement quantique, qui mesure le rapport entre l'intensité émise et l'intensité d'excitation, permet de classer ces maxima selon leur efficacité. L'efficacité maximale est obtenue pour une excitation à 310 nm; le rendement est plus faible, mais encore très voisin, pour 375 nm; en revanche il est très inférieur pour la radiation à 260 nm.

Mais les propriétés usuelles d'absorption lumineuse présentées par les fibres optiques font qu'il n'était pas possible d'envisager d'utiliser l'excitation de la fluorescence à 260 nm pour une utilisation avec endoscope. De même on ne pouvait envisager d'utiliser la longueur d'onde de 310 nm, car les verres employés dans les appareillages de détection de fluorescence ne sont transparents qu'au-delà de 360 nm. Aussi est-ce grâce à la mise en évidence d'une longueur d'onde d'excitation efficace à 375 nm que l'on a pu envisager une telle application de révélation par fluorescence.

On a au surplus pu établir que, à intensité d'excitation constante, il existe une relation linéaire entre l'intensité de fluorescence et la concentration en alstonine. Cela s'est vérifié tout particulièrement dans un domaine de concentration en alstonine suffisant pour la détection de l'alstonine, mais suffisamment faible pour que soient minimisés les risques de toxicité du produit vis-à-vis des cellules saines.

L'invention est décrite plus en détail ci-après en référence aux figures annexées, dans lesquelles:
- Fig. 1 représente les spectres d'excitation et d'émission de l'alstonine;
- Fig. 2 représente le spectre d'absorption de l'alstonine;
- Fig. 3 A représente la variation de l'intensité de fluorescence en fonction de la concentration en alstonine; Fig. 3 B et Fig. 3 C sont des tracés montrant que cette variation est linéaire dans le domaine de concentration en alstonine de 10⁻⁶ à 10⁻⁵;
- Fig. 4 A à 4 D montrent les résultats de l'analyse de quelques grandeurs physico-chimiques de l'alstonine mise à incuber selon l'invention dans deux populations cellulaires SK0 et ST3, effectuée au moyen d'un analyseur d'image par fluorescence;
- Fig. 5 A à 5 C représentent schématiquement les résultats d'une chromatographie sur gel de Sephadex, avec élution avec du tampon tris-HCl 10 mM, pH 7,5.

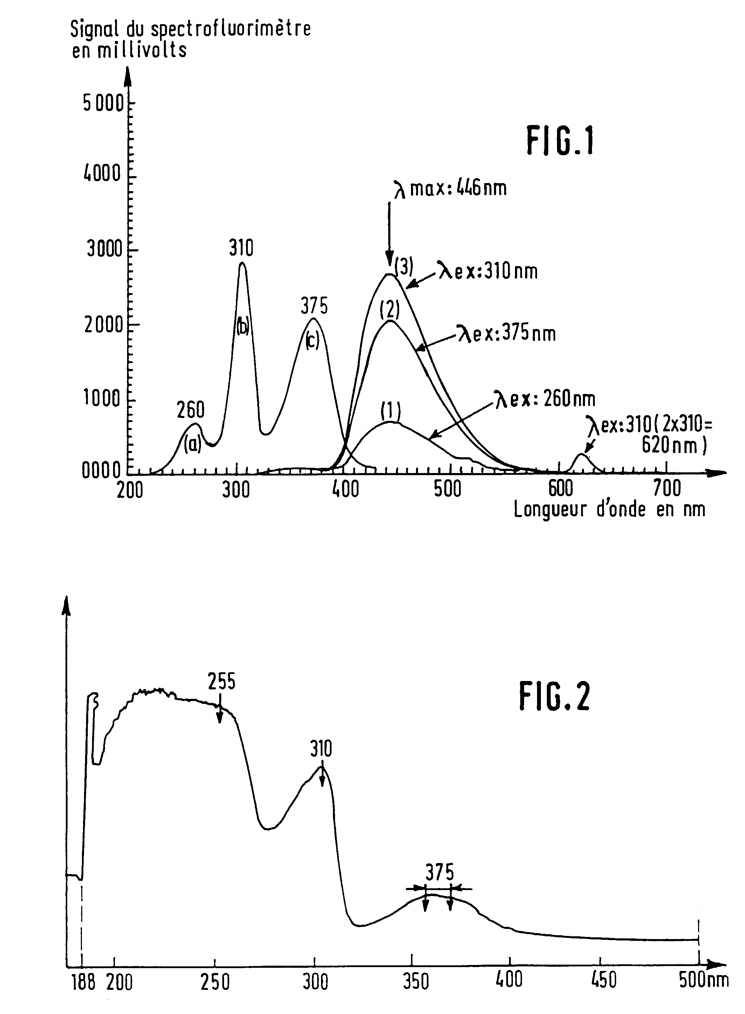

Comme il a été dit plus haut, le spectre de fluorescence de l'alstonine est centré sur la longueur d'onde de 446 nm (Fig. 1), tandis que le spectre d'excitation de la fluorescence (Fig. 1) et le spectre d'absorption (Fig. 2) se sont avérés présenter trois maxima correspondant respectivement aux longueurs d'onde de 252 nm, 310 nm et 375 nm.

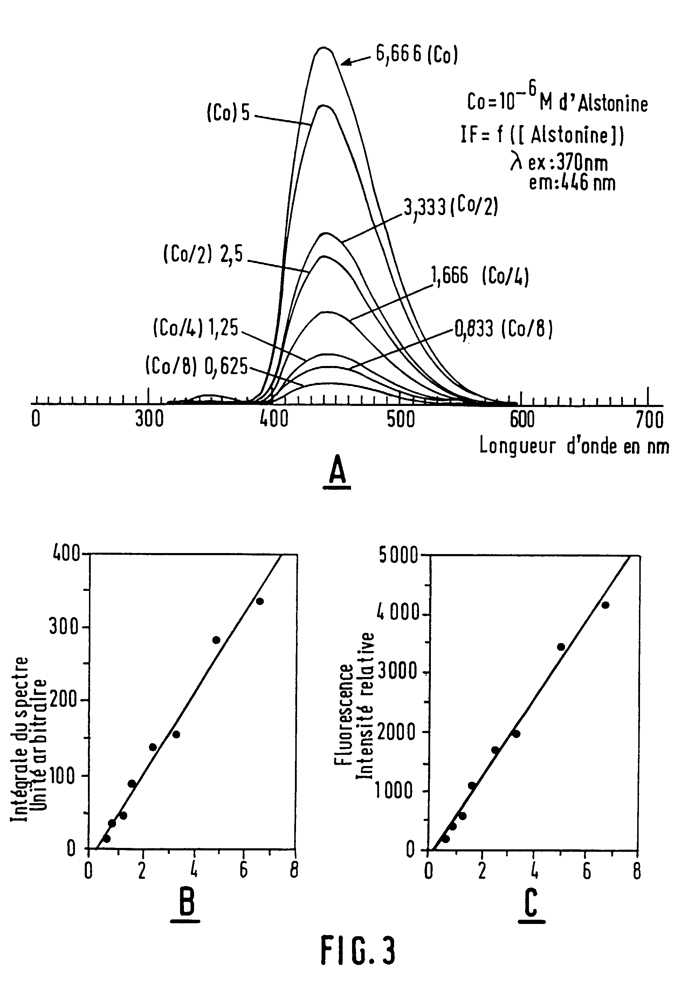

Ainsi que le montrent les résultats consignés sur les figures 3 A à 3 C, l'intensité de fluorescence de l'alstonine et la concentration en cet alcaloïde sont dans une relation linéraire entre elles, à intensité d'excitation constante, dans un domaine de concentration en alstonine approprié pour la détection par les appareillages classiques en spectrofluorimétrie.

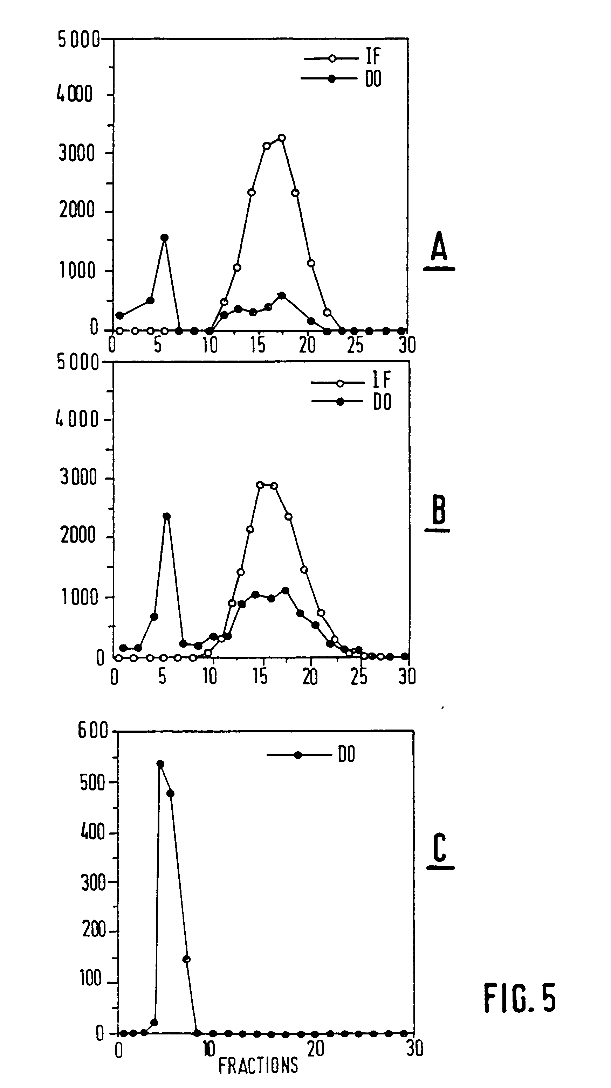

Par ailleurs des études effectuées par chromatographie sur colonnes de gel de Sephadex (Fig. 5 A à FiG 5 C) ont montré qu'il n'y a aucune interaction entre l'ADN double brin ou l'ADN simple brin isolés des cellules normales et l'alstonine. Ces résultats sont en accord avec les publications sur l'alstonine précisant que celle-ci ne s'accumule que dans les cellules tumorales en raison de son interaction avec l'ADN tumoral [voir en particulier à ce sujet: M. Beljanski et M.S. Beljanski, Exp. Cell. Biol., S. Karger AG, Bâle, Pub. 50: 79-87 (1982) et M. Beljanski et M.S. Beljanski, Oncology, S. karger AG, Bâle, Pub. 43: 198-203 (1986)].

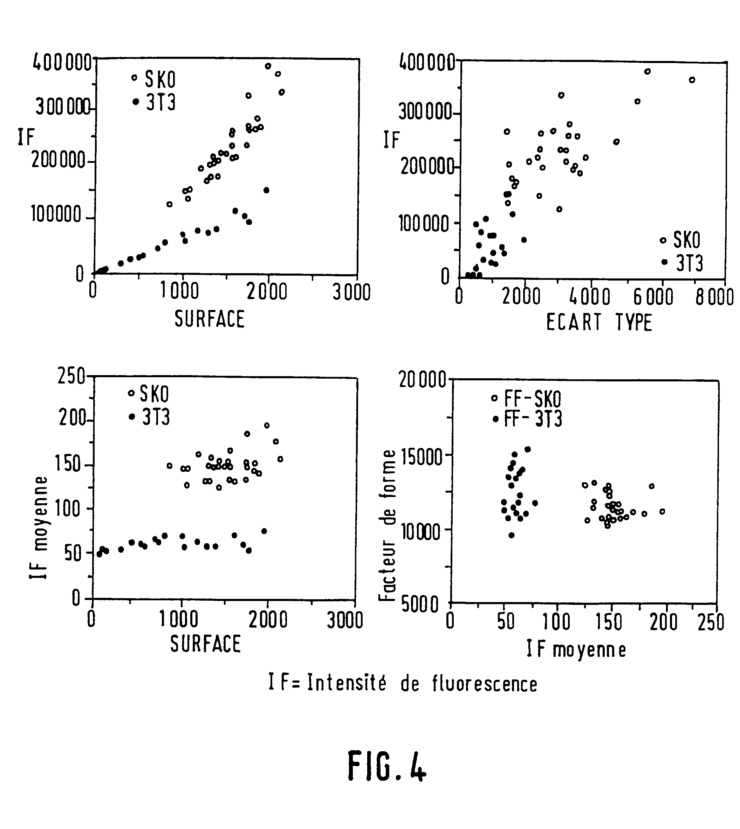

Quant aux études d'accumulation de l'alstonine réalisées sur des cellules vivantes en culture soit normales (ST3) soit tumorales (SK0), elles montrent (Fig. 4 A à 4 D) que, après une incubation d'environ 20 minutes ou plus en présence du produit, les études de fluorescence conduites avec un analyseur d'image Samba 2000 révèlent une accumulation importante uniquement dans les cellules SK0.

Pour la mise en oeuvre des moyens et de la méthode selon la présente invention, il convient dans la pratique d'utiliser de l'alstonine en solution aqueuse à raison d'environ 0,2 à 5 mg/20 ml d'eau, soit à une concentration d'environ 1 à 25% en poids/volume, dans les applications in vitro..

Pour la constitution d'un kit ou trousse de diagnostic utilisable principalement dans des tests in vitro, il est recommandé d'utiliser comme matière première une solution aqueuse purifiée d'alstonine à saturation et d'en effectuer extemporanément des dilutions à l'eau pure pour analyses à des concentrations utiles, variant en pratique entre environ 5 et 10% en poids/volume.

In vivo il convient en pratique d'administrer l'alstonine sous une forme galénique appropriée, comportant les supports ou vecteurs pharmaceutiques classiques adaptés et des excipients éventuels, telle que par exemple des comprimés ou des gélules, avantageusement à raison d'environ 3 à 500 mg d'alstonine par unité de dosage, l'administration étant faite par voie orale, trois fois par jour, la veille de l'exploration diagnostique in vivo.

L'incubation est avantageusement effectuée sur une durée de 20 à 40 minutes environ, à température ambiante.

Dans les applications in vitro, une fois cette étape d'incubation terminée, on rince l'échantillon à l'alcool, de préférence au méthanol, et on effectue une lecture au microscope à fluorescence.

Pour une utilisation clinique de la méthode et des moyens selon l'invention, notament dans le diagnostic pré-, per- et/ou post-opératoire et dans le diagnostic de maladies cutanées en confirmation d'autres examens, il convient de pratiquer une révélation de la fluorescence sous excitation à la lampe de Wood. Cette bioluminescence à la lampe de Wood est utilisée soit par illumination directe, soit par transmission, notamment par des fibres optiques, dans les cas d'explorations endoscopiques.

En variante, une composition à l'alstonine selon l'invention peut aussi avantageusement être utilisée en cytogénétique, pour la recherche notamment d'une identification chromosomique aberrante, par la mise en évidence au niveau chromosomique de cassures, de remaniements et/ou de recombinaisons. Dans la pratique cette identification d'aberration chromosomique, caractéristique d'une maladie génétique héréditaire, s'opère sous la forme d'une bande de luminescence spécifique d'un ou de plusieurs chromosomes impliqués dans ladite aberration chromosomique et révélée par la technique selon la présente invention.

A titre d'exemple, des études diagnostiques s'appuyant sur une composition d'alstonine conforme à l'invention, selon des protocoles d'applications biologiques appropriés, ont permis de vérifier du point de vue cytogénétique les corrélations entre la perte d'un des deux gonosomes et l'installation de tumeurs solides ou hémopathies malignes:
- dans le cas des sujets féminins, le caryotype devenant alors 45 X 0 par la perte d'un chromosome X,
- dans le cas des sujets masculins, le caryotype devenant alors 45 X 0, par la perte du chromosome Y.

Le mécanisme d'action cytogénétique qui contrôle ces événements peut ainsi être apprécié qualitativement et quantitativement par des études différentielles de coloration et de culture lymphocytaire, permettant de mettre en évidence les ponts et cassures et les recombinaisons.

Bien que la description qui précède et les exemples illustratifs ci-après fassent référence à l'alstonine en tant qu'alcaloïde particulier, l'homme du métier est à même de concevoir aisément que des résultats comparables peuvent être obtenus si l'on substitue totalement ou partiellement à cet alcaloïde un quelconque équivalent technique de celui-ci pour l'application considérée, notamment son isomère la serpentine.

### Exemples d'utilisation pour un diagnostic sur prélèvements biologiques

### Exemple 1

On a imprégné avec une solution aqueuse d'alstonine contenant environ 200 microgrammes d'alstonine par millilitre divers prélèvements biologiques, qui ont été ensuite incubés pendant 20-40 minutes, puis un examen microscopique sous microscope à lampe de Wood a été effectué.

On a ainsi traité et examiné un étalement de prélèvement de col utérin sur lame de verre et mis en évidence par la bioluminescence observée sous microscope à lampe de Wood la nature cancéreuse des éléments cellulaires.

### Exemple 2

On a procédé comme indiqué dans l'exemple 1 sur un étalement de moelle osseuse sur lame de verre.

La bioluminescence observée sous microscope à lampe de Wood a révélé la nature cancéreuse des éléments cellulaires.

### Exemple 3

En procédant comme indiqué dans l'exemple 1 avec un prélèvement gastrique, après centrifugation et étalement sur lame de verre, on a mis en évidence la présence de cellules tumorales par la bioluminescence de l'étalement sur lame révélée par la méthode sélective selon l'invention.

### Exemple 4

En procédant comme indiqué dans l'exemple 1 avec un prélèvement vésical étalé sur lame de verre, on a mis en évidence la présence de cellules tumorales par la bioluminescence de l'étalement sur lame révélée par la méthode sélective selon l'invention.

Les moyens et la méthode selon la présente invention sont également utiles pour la révélation en cytogénétique in vitro des aberrations chromosomiques liées à une maladie génétique héréditaire, cette révélation s'opérant par l'identification d'une bande de luminescence spécifique d'un ou de plusieurs chromosomes impliqués dans des cassures, remaniements ou recombinaisons.

### Exemples d'utilisation clinique

### Exemple 5

On a administré par voie orale, trois fois à des intervalles sensiblement équivalents sur une journée, la veille de l'exploration diagnostique, une gélule dosée à 250 mg d'alstonine, préparé comme indiqué plus haut, à des patientes chez lesquelles devaient être recherchées et évaluées des tumeurs ovariennes ou vésicales. Une observation par endoscopie effectuée le jour suivant l'administration des gélules, avec transmission à travers l'endoscope des rayonnements d'excitation en UV générés par une lampe de Wood, a permis de vérifier que la détection des tumeurs néoplasiques était aisée par ce moyen. On a également pu établir que le diagnostic différentiel avec des tumeurs cancéreuses était confirmé par prélèvement biopsique et examen anatomopathologique.

### Exemple 6

Selon le mode opératoire indiqué dans l'exemple 5, un diagnostic de pancréatite avec évolution en vascularite extra-pancréatique de type weber-Christian a été effectué chez un malade, Mr. A.G., présentant une évolution torpide depuis 10 ans. On a ainsi pu vérifier significativement que la consolidation était effective après ces dix années.

### Exemple 7

Selon le mode opératoire indiqué dans l'exemple 5, le diagnostic d'une tumeur de Merkel a été confirmé chez une femme de 74 ans; ce diagnostic a également été confirmé par des analyses histologiques de l'exérèse d'une tumeur du type carcinome neuro-endocrinien de type APUD.

### Exemple 8

Selon le mode opératoire indiqué dans l'exemple 5, une ichtyose généralisée a été détectée sous UV chez le jeune S.L., âgé de 8 ans, et il a ainsi été constaté l'existence d'une kératose épaisse sans oligophrénie. En répétant ultérieurement l'investigation selon l'invention, on a vérifié l'amélioration résultant du traitement, se traduisant par un décapage du visage, des membres inférieurs et des membres supérieurs, mais avec persistance de l'ichtyose au niveau du cuir chevelu, de l'abdomen, du thorax et du dos.

### Exemple 9

Selon le mode opératoire indiqué dans l'exemple 5, une épithéliomatose du visage de type sénile avec évolution vers une formation baso-cellulaire frontale droite a été analysée et localisée chez un patient. En répétant les investigations conformément à l'invention à des intervalles de temps appropriés, on a pu suivre les effets du traitement appliqué, jusqu'au comblement de la formation baso-cellulaire et à sa cicatrisation.

### Exemple 10

Le mode opératoire indiqué dans l'exemple 5 a été appliqué à un patient, Mr. C.Y., présentant un lymphome cutané du type maladie de Sésary. Celui-ci a montré sous exploration UV après l'administration d'alstonine dans les conditions indiquées à l'exemple 1 une infiltration lymphoïde du visage de type léonin, ainsi que des zones de dermo-épidermite et des pseudo-plaques de leucodysplasie du visage et des lèvres.

### Exemple 11

Chez un patient, Mr. D.F., sur lequel il a été opéré comme indiqué dans l'exemple 5, l'effet marqueur de l'alstonine sous UV a montré l'existence d'une récidive de fibrosarcome, après échec de son traitement thérapeutique à une date antérieure de cinq ans à cette observation sous UV. Le contrôle du traitement par de l'alstonine sous UV a montré la disparition de la luminescence cicatricielle, ce qui témoignait de la récupération du tissu normal au niveau de la tumeur.

### Exemple 12

Selon le mode opératoire indiqué dans l'exemple 5, l'existence d'un adéno-carcinome recto-sigmoïdien a été établie et visualisée par la fluorescence de l'alstonine chez un patient, Mr. K., pour lequel ce diagnostic a été confirmé par l'étude anatomopathologique de l'exérèse d'une récidive d'une lésion villeuse montrant en particulier la présence d'un foyer prolifératif adéno-carcinomateux de stade I.

### Exemple 13

En opérant comme indiqué dans l'exemple 5 et après gastréctomie totale sur un patient, Mr. A.J., en raison de l'existence au niveau de la petite courbure gastrique et de la chaîne ganglionnaire coéliaque d'une masse néoplasique, on a pu sous la bioluminescence de la pièce opératoire faire la topographie des zones hyperluminescentes, correspondant aux sites des ulcérations néoplasiques de la petite courbure verticale.

### Exemple 14

En opérant comme indiqué dans l'exemple 5 sur une patiente, Mme E.Y., sur laquelle a ensuite été opérée une laparotomie après contrôle échographique, on a pu voir, grâce à la bioluminescence de l'alstonine après laparotomie, l'existence d'une luminescence importante sur un ovaire et vérifier qu'il s'agissait en fait d'une prolifération métastatique d'une tumeur primaire du sein.

### Exemple 15

On a opéré comme indiqué dans l'exemple 5 chez un patient, Mr. G.M., traité par cobaltothérapie de contact pour une tumeur spino-cellulaire de l'aile du nez. La bioluminescence de l'alstonine a permis de mettre en évidence la récidive et des modifications dysplasiques naso-orbitales du côté opposé (effet ricochet).

Des essais comparables menés avec les autres bêta-carbolines ayant des caractéristiques équivalentes de fluorescence, à savoir principalement la serpentine, ont donné des résultats analogues et constituent donc des équivalents techniques de l'alstonine dans le cadre de la présente invention.

Les moyens selon l'invention sont donc remarquablement utiles pour le cytodiagnostic, qui comprend l'évaluation des affections tumorales et la cytogénétique. In vivo, ils permettent, avec une précision et une facilité jamais atteintes à ce jour, au stade préopératoire de localiser une tumeur avant opération; au stade peropératoire de suivre grâce à la fluorescence l'étude des lésions cancéreuses et des métastases; au stade postopératoire de vérifier par endoscopie, en utilisant des fibres optiques, si des métastases apparaissent ou progressent postérieurement à un traitement anticancéreux ou à une chimiothérapie par des méthodes traditionnelles.

## Revendications

1. Utilisation d'une composition d'alstonine et/ou d'un isomère de celle-ci pour l'obtention d'un réactif de cytodiagnostic destiné à la révélation sélective des affections tumorales et/ou à la révélation in vitro des aberrations chromosomiques liées à une maladie génétique héréditaire.

2. Utilisation d'une composition d'alstonine et/ou d'un isomère de celle-ci selon la revendication 1, caractérisée en ce que ladite composition comporte de l'alstonine et/ou un isomère de celle-ci en solution aqueuse à raison d'environ 0,2 à 5 mg/20 ml d'eau, soit à une concentration d'environ 1 à 25% en poids/volume.

3. Utilisation d'une composition d'alstonine et/ou d'un isomère de celle-ci pour utilisation selon la revendication 1, caractérisée en ce que ladite composition consiste en une solution aqueuse purifiée d'alstonine et/ou d'un isomère de celle-ci à saturation, pour dilution extemporanée à des concentrations utiles variant entre environ 5 et 10% en poids/volume.

4. Utilisation d'une composition d'alstonine et/ou d'un isomère de celle-ci selon la revendication 1, caractérisée en ce que ladite composition est sous forme de comprimés ou de gélules, comprenant de préférence environ 3 à 500 mg d'alstonine et/ou d'un isomère de celle-ci par unité de dosage, ainsi que des supports ou vecteurs pharmaceutiques classiques et des excipients habituels.

5. Utilisation d'une composition d'alstonine et/ou d'un isomère de celle-ci selon l'une quelconque des revendications 2 à 4, caractérisée en ce que ladite composition comporte en outre de la serpentine.

6. Méthode d'évaluation des tumeurs et/ou de cytogénétique in vitro, utile pour le diagnostic des affections tumorales et des aberrations chromosomiques, caractérisée en ce qu'elle comprend la révélation des cellules tumorales par fluorescence sélective détectée au moyen d'une activation par une lampe de wood émettant à environ 375 nm ou d'une détection au microscope à fluorescence à environ 375 nm, après fixation sélective d'une quantité appropriée d'une composition pharmaceutique selon l'une quelconque des revendications 2 à 5 et incubation.

7. Méthode selon la revendication 6, caractérisée en ce qu'elle comprend l'imprégnation de l'échantillon avec une solution aqueuse comportant par ml environ 200 Mg d'alstonine et/ou d'un isomère de celle-ci et l'incubation pendant 20-40 minutes à température ambiante.

8. Méthode selon la revendication 6, caractérisée en ce qu'on effectue la révélation par au moins une mesure de fluorescence à environ 446 nm pour révélation d'une excitation à environ 375 nm.

9. Kit ou trousse pour faciliter le diagnostic des affections tumorales et/ou des aberrations chromosomiques, caractérisé en ce qu'il comporte une composition pour utilisation selon l'une quelconque des revendications 1 à 5 et telle que définie dans lesdites revendications.

## Patentansprüche

1. Verwendung einer Zusammensetzung von Alstonin und/oder eines Isomers hiervon zur Herstellung eines zytodiagnostischen Mittels zum selektiven Nachweis von Tumorkrankheiten und/oder zum *in vitro*-Nachweis von chromosomalen Aberrationen, die verknüpft sind mit einer erblichen genetischen Krankheit.

2. Verwendung einer Zusammensetzung von Alstonin und/oder eines Isomers hiervon zur Verwendung gemäß Anspruch 1, dadurch gekennzeichnet, daß die Zusammensetzung umfaßt Alstonin und/oder ein Isomer hiervon in einer wäßrigen Lösung in einer Konzentration von etwa 0,2 bis 5 mg pro 20 ml Wasser, d.h. einer Konzentration von etwa 1 bis 25% Gewicht/Volumen.

3. Verwendung einer Zusammensetzung von Alstonin und/oder eines Isomers hiervon zur Verwendung gemäß Anspruch 1, dadurch gekennzeichnet, daß die Zusammensetzung besteht aus einer gesättigten gereinigten wäßrigen Lösung von Alstonin und/oder einem Isomer hiervon, die verdünnt wird zum Zeitpunkt der Verwendung auf nützliche Konzentrationen in einem Bereich von etwa 5 bis 10% Gewicht/Volumen.

4. Verwendung einer Zusammensetzung von Alstonin und/oder eines Isomers hiervon zur Verwendung gemäß Anspruch 1, dadurch gekennzeichnet, daß die Zusammensetzung in Form von Tabletten oder Kapseln ist, bevorzugt enthaltend etwa 3 bis 500 mg Alstonin und/oder einem Isomer hiervon pro Dosierungseinheit sowie übliche pharmazeutische Träger oder Zusatzstoffe.

5. Verwendung einer Zusammensetzung von Alstonin und/oder eines Isomers hiervon gemäß einem der Ansprüche 2 bis 4, dadurch gekennzeichnet, daß die Zusammensetzung Serpentin umfaßt.

6. Verfahren zur *in vitro*-Evaluierung von Tumoren und/oder der Zytogenese zur Diagnose von Tumorkrankheiten und chromosomalen Aberrationen, dadurch gekennzeichnet, daß es den Nachweis von Tumorzellen durch selektive Fluoreszenz, nachgewiesen durch Aktivierung mit einer Wood's Lampe mit Licht einer Wellenlänge von etwa 375 nm oder durch Fluoreszenzmikroskopie bei etwa 375 nm nach selektiver Fixierung mit einer geeigneten Menge einer pharmazeutischen Zusammensetzung gemäß einem der Ansprüche 2 bis 5 und Inkubierung umfaßt.

7. Verfahren gemäß Anspruch 6, dadurch gekennzeichnet, daß es die Imrägnierung der Probe mit einer wäßrigen Lösung mit etwa 200 µg von Alstonin und/oder eines Isomers hiervon pro ml und Inkubierung für 20 bis 40 Minuten bei Umgebungstemperatur umfaßt.

8. Verfahren gemäß Anspruch 6, dadurch gekennzeichnet, daß der Nachweis ausgeführt wird durch Messen der Fluoreszenz bei etwa 446 nm zum Nachweis der Anregung bei etwa 375 nm.

9. Kit zur Erleichterung der Diagnose von Tumorkrankheiten und/oder chromosomalen Aberrationen, dadurch gekennzeichnet, daß er eine Zusammensetzung zur Verwendung gemäß einem der Ansprüche 1 bis 5 und wie in diesen Ansprüchen definiert, umfaßt.

## Claims

1. Use of a composition of alstonine and/or of an isomer thereof for the preparation of a cytodiagnostic agent designed for the selective detection of tumoral diseases and/or for the *in vitro* detection of chromosomal aberrations linked with a hereditary genetic disease.

2. Use of a composition of alstonine and/or of an isomer thereof according to claim 1, characterized in that said composition comprises alstonine and/or an isomer thereof in an aqueous solution at a concentration of about 0.2 to 5 mg per 20 ml of water, i.e. a concentration of about 1 to 25% by weight/volume.

3. Use of a composition of alstonine and/or of an isomer thereof for use according to claim 1, characterized in that said composition consists in a purified saturated aqueous solution of alstonine and/or of an isomer thereof, to be diluted, at the time of use, to useful concentrations which range between about 5 and 10% by weight/volume.

4. Use of a composition of alstonine and/or of an isomer thereof according to claim 1, characterized in that said composition is in the form of tablets or capsules, preferably containing about 3 to 500 mg of alstonine and/or of an isomer thereof per unit dose, as well as conventional pharmaceutical supports or carriers and usual excipients.

5. Use of a composition of alstonine and/or of an isomer thereof according to any one of claims 2 to 4, characterized in that said composition also comprises serpentine.

6. Method for the *in vitro* evaluation of tumors and/or for cytogenetics, useful for the diagnosis of tumoral diseases and chromosomal aberrations, characterized in that it comprises the detection of tumor cells by selective fluorescence detected by activation with a Wood's lamp which emits at about 375 nm or by detection by fluorescence microscopy at about 375 nm, after selective fixation of a suitable amount of a pharmaceutical composition according to any one of claims 2 to 5, and incubation.

7. Method according to claim 6, characterized in that it comprises impregnating the sample with an aqueous solution containing about 200 µg of alstonine and/or of an isomer thereof per ml and incubating for 20-40 minutes at ambient temperature.

8. Method according to claim 6, characterized in that the detection is effected by means of at least one measurement of fluorescence at about 446 nm for the detection of an excitation at about 375 nm.

9. Kit for facilitating the diagnosis of tumoral diseases and/or of chromosomal aberrations, characterized in that it comprises a composition for use according to any one of claims 1 to 5 and such as defined in the said claims.
